# EUROPÄISCHE PATENTSCHRIFT

(11) **EP 1 078 973 B1**
(45) Veröffentlichungstag und Bekanntmachung des Hinweises auf die Patenterteilung: **27.11.2002**
(21) Anmeldenummer: 00117696.5
(22) Anmeldetag: 17.08.2000
(51) Int. Cl.: C09K 15/20, C07C 7/20, C07B 63/04

(54) **Stabilisierte Monomerzusammensetzung**
Stabilised monomer composition
Composition de monomère stabilisée

(30) Priorität: 27.08.1999 DE 19940623
(43) Veröffentlichungstag der Anmeldung: 28.02.2001
(73) Patentinhaber: Röhm GmbH & Co. KG, 64293 Darmstadt (DE)
(72) Erfinder: Scharf, Jakob, 67549 Worms (DE); Rau, Hartmut, 67574 Osthofen (DE); Götzen, Friedrich, Dr., 67550 Worms (DE)

(56) Entgegenhaltungen:
- EP-A- 0 266 906
- EP-A- 0 467 850
- EP-A- 0 924 228
- WO-A-89/10343
- US-A- 4 956 020

## Beschreibung

Die vorliegende Erfindung betrifft stabilisierte Monomerzusammensetzungen sowie Verfahren zu deren Herstellung.

Die Stabilisierung von Monomerzusammensetzungen ist ein weithin bekanntes Problem. So zeigen beispielsweise insbesondere Derivate der Methacrylsäure unerwünschte, vorzeitige Polymerisation. Speziell bei erhöhten Temperaturen steigt die Polymerisationsgeschwindigkeit stark an. Viele Verfahren zur Herstellung von acrylischen Monomeren umfassen typischerweise jedoch Prozeßschritte, wie beispielsweise Destillationen, bei denen die Temperatur erhöht wird. Um diese Reinigungsprozesse dennoch durchführen zu können, ist die Stabilisierung der zu reinigenden Monomere notwendig. Auch eine längerfristige Lagerung führt dazu, daß Polymere entstehen. Insbesondere bei Lichteinwirkung treten diese Alterungserscheinungen auf.

Es ist zwar möglich, diese Polymerisation durch Zugabe von hohen Mengen an bekannten Inhibitoren zu vermindern. Dies führt jedoch dazu, daß eine erwünschte Polymerisation anschließend nur durch Zugabe von entsprechend großen Mengen an Initiatoren erreicht werden kann. Bei einer sehr großen Menge an zugegebenem Inhibitor muß dieser vor einer Polymerisation jedoch abgetrennt werden, da andernfalls hohe Molekulargewichte verhindert werden. Aus diesem Grunde sollten möglichst geringe Inhibitorkonzentrationen eingesetzt werden.

Bekannte Polymerisationsinhibitoren für Acrylate umfassen beispielsweise Phenothiazin, Hydrochinon, insbesondere Hydrochinonmonomethylether und Benzochinon. Des weiteren ist auch die Verwendung von N,N-Diethylhydroxylamin bekannt. So offenbart z.B. die europäische Patentanmeldung EP-A-0 266 906 die synergistiche Wirksamkeit von Hydroxylaminen in Kombination mit Phenylendiaminen. Es hat sich jedoch gezeigt, daß diese Kombination in Verfahren zur Herstellung von Hydroxyalkyl(meth)acrylaten allein unzureichend ist. So treten insbesondere bei der destillativen Aufreinigung Ausbeuteverluste auf.

In Anbetracht des hierin angegebenen und diskutierten Standes der Technik war es Aufgabe der vorliegenden Erfindung, eine stabilisierte Monomerzusammensetzung anzugeben, die über lange Zeit lagerfähig ist, ohne daß der vorhandene Inhibitor nicht mehr durch Zugabe von Initiatoren überfahren werden könnte, also auch hohe Polymerisationsgrade ohne Abtrennung des Inhibitors erhalten werden können.

Eine weitere Aufgabe der Erfindung bestand darin, eine stabilisierte Monomerzusammensetzung aufzufinden, die besonders zur Destillation geeignet ist.

Des weiteren lag der Erfindung die Aufgabe zugrunde, Zusammensetzungen zu finden, die eine hohe Produktausbeute bei der Herstellung von Hydroxyalkyl(meth)acrylaten ermöglichen.

Gelöst werden diese Aufgaben durch stabilisierte Monomerzusammensetzungen mit den Merkmalen des Anspruchs 1. So sind Gegenstand der Erfindung stabilisierte Monomerzusammensetzungen, die mindestens ein ethylenisch ungesättigtes Monomer und eine stabilisierend wirkende Menge einer Kombination von N,N-Diethylhydroxylamin und N-Nitroso-N-Phenylhydroxylamin aufweisen.

Vorteilhafte Ausgestaltungen der erfindungsgemäßen Monomerzusammensetzungen sind Gegenstand der rückbezogenen Ansprüche. So können die erfindungsgemäßen Monomerzusammensetzungen insbesondere weitere allgemein bekannte Inhibitoren und Antioxidatien umfassen. Hinsichtlich des Verfahrens zu Herstellung liefert Anspruch 9 die Lösung der zugrundeliegenden Aufgabe.

Durch die erfindungsgemäßen Maßnahmen werden u.a. insbesondere folgende Vorteile erzielt:
⇒ Erfindungsgemäß stabilisierte Monomerzusammensetzungen sind auch bei Lichteinwirkung langzeitlagerfähig.
⇒ Monomere, die gemäß der vorliegenden Erfindung stabilisiert wurden, können besonders leicht durch Destillation aufgereinigt werden.
⇒ Stabilisierte Monomerzusammensetzungen können des weiteren durch bekannte Reaktionen umgesetzt werden, in denen das Monomer unter Erhalt der ethylenisch ungesättigten Doppelbindung verändert wird, ohne daß diese Umsetzung zu besonders hohen Ausbeuteverlusten führt.
⇒ Ganz besonders überraschend ist, daß durch die erfindungsgemäße Kombination auch Monomere stabilisiert werden, die sich in der Gasphase befinden.

Ethylenisch ungesättigte Monomere sind Verbindungen, die zumindest eine radikalisch polymerisierbare Doppelbindung aufweisen. Zu diesen ethylenisch ungesättigten Monomeren gehören u.a. Vinylester, (Meth)acrylsäure, Ester der (Meth)acrylsäure, beispielsweise Methyl- und Ethyl (meth)acrylat, Vinylchlorid, Vinylidenchlorid, Vinylacetat, Styrol, substituierte Styrole mit einem Alkylsubstituenten in der Seitenkette, wie z.B. α-Methylstyrol und α-Ethylstyrol, substituierte Styrole mit einem Alkylsubstituenten am Ring, wie beispielsweise Vinyltoluol und p-Methylstyrol, halogenierte Styrole, wie beispielsweise Monochlorstyrole, Dichlorstyrole, Tribromstyrole und Tetrabromstyrole, Vinyl- und Isopropenylether, Maleinsäurederivate, wie beispielsweise Maleinsäureanhydrid, Methylmaleinsäureanhydrid, Maleinimid, Methylmaleinimid, und Diene, wie beispielsweise Divinylbenzol.

Die Schreibweise (Meth)acryl umfaßt Methacryl, Acryl und Mischungen aus beiden.

Bevorzugte ethylenisch ungesättigte Monomere sind (Meth)acrylsäure sowie Derivate der (Meth)acrylsäure. Diese Verbindungen können beispielsweise gemäß Formel 1 dargestellt werden, 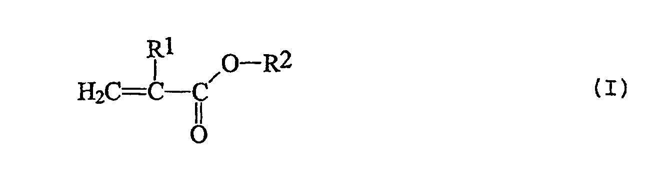 worin der Rest R¹ Wasserstoff oder eine Methylgruppe, der Rest R² Wasserstoff, einen Arylrest, der auch Heteroatome enthalten kann, wie beispielsweise Phenyl und Imidazol, sowie einen gradkettigen, verzweigten oder cyclischen Alkylrest mit bis zu 30 Kohlenstoffatomen darstellt, der sowohl gesättigt als auch ungesättigt sein kann, und auch Heteroatome, wie beispielsweise Stickstoff und/oder Sauerstoff enthalten kann, wie beispielsweise Methyl, Ethyl,-Propyl, Butyl, Pentyl, Hexyl, Cyclopentyl, Cyclohexyl, Isobornyl, Vinyl, Propenyl, Butinyl, 2-(N,N-Dimethylamino)ethyl, 2-(N,N-Dimethylamino)propyl, 2-Hydroxypropyl und 2-Hydroxyethyl.

Zu diesen Verbindungen gehören u.a. Methyl (meth) acrylat, Ethyl (meth) acrylat, Propyl(meth) acrylat, Isopropyl(meth)acrylat, n-Butyl(meth)acrylat, Isobornyl(meth)acrylat, Hydroxylalkyl (meth)acrylate, wie 2-Hydroxyethyl(meth)acrylat, 2-Hydroxypropyl(meth)acrylat, 3-Hydroxypropyl(meth)acrylat und 3,4-Dihydroxybutyl(meth)acrylat, sowie Aminoalkyl(meth)acrylate, wie Dimethylaminoethylmethacrylat (DMAEMA).

Die den (Meth)acrylsäureestern entsprechenden (Meth)acrylsäureamide sind ebenfalls eine bevorzugte Gruppe von ethylenisch ungesättigten Monomeren. Hierzu gehört beispielsweise N-Dimethylaminopropylmethacrylamid (DMAPMA).

Die ethylenisch ungesättigten Monomere können einzeln oder als Mischung in der erfindungsgemäßen Zusammensetzung vorhanden sein.

Die Verbindungen N,N-Diethylhydroxylamin und N-Nitroso-N-Phenylhydroxylamin ("Kupferron") sind in der Fachwelt weithin bekannt und von einer Vielzahl von Anbietern kommerziell erhältlich. N-Nitroso-N-Phenylhydroxylamin ist eine saure Verbindung, wobei deren Salze, wie beispielsweise das Ammonium-, Aluminium-, Kupfer-, Lithium-, Natrium-, Kalium- und Rubidiumsalz, ebenfalls von der vorliegenden Erfindung umfaßt werden sollen.

Vorzugsweise enthalten die erfindungsgemäßen Zusammensetzungen die Komponenten N,N-Diethylhydroxylamin und N-Nitroso-N-Phenylhydroxylamin in einer synergistischen Menge.

Synergismus bedeutet hierbei, daß die inhibierende Wirkung der Kombination größer ist als die Inhibierung die aufgrund der Wirksamkeit der einzelnen Verbindungen erwartet werden kann.

Vorzugsweise liegt das Verhältnis von N,N-Diethylhydroxylamin zu N-Nitroso-N-Phenylhydroxylamin, bezogen auf das Gewicht, im Bereich von 1:1 bis 10:1. Die Menge des N,N-Diethylhydroxylamins liegt vorzugsweise im Bereich von 10 bis 500 ppm, wobei das N-Nitroso-N-Phenylhydroxylamin bevorzugt in einer Menge im Bereich von 10 bis 500 ppm vorhanden ist, jeweils bezogen auf das Gesamtgewicht der Zusammensetzung.

Der untere Grenzwert ergibt sich aus der Lagerfähigkeit der stabilisierten Monomerzusammensetzung. Werden weniger als 10 ppm der jeweiligen stabilisierend wirkenden Verbindung eingesetzt, so ist die Lagerfähigkeit für viele Zwecke unzureichend. Falls nur eine relativ geringe Stabilisierung der Monomerzusammensetzung erwünscht ist, können auch geringere Mengen eingesetzt werden.

Der obere Grenzwert ergibt sich einerseits aus wirtschaftlichen Gesichtspunkte, andererseits sollte eine erwünschte Polymerisation, die durch Initiatoren gestartet wird, möglich sein. Dementsprechend hat sich der obere Grenzwert als eine vorteilhafte Ausgestaltung erwiesen, ohne daß hierdurch eine Beschränkung erfolgen soll.

Die erfindungsgemäße Zusammensetzung kann weitere Inhibitoren aufweisen. Diese sind in der Fachwelt weithin bekannt. So können beispielsweise 1,4-Dihydroxybenzole zur Stabilisierung zusätzlich zugegeben werden. Es können jedoch auch anders substituierte Dihydroxybenzole zum Einsatz kommen. Allgemein lassen sich derartige Inhibitoren mit der allgemeinen Formel (II) wiedergeben 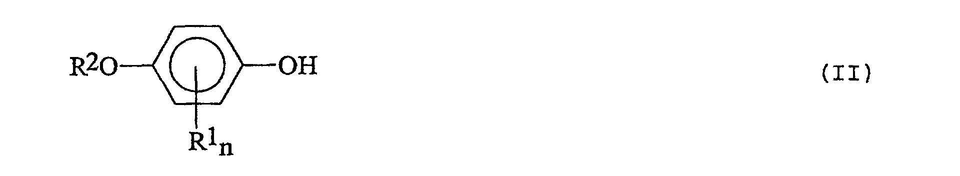 worin
R¹ einen linearen oder verzweigten Alkylrest mit eins bis acht Kohlenstoffatomen, Halogen oder Aryl bedeutet, vorzugsweise einen Alkylrest mit eins bis vier Kohlenstoffatomen, besonders bevorzugt Methyl, Ethyl, n-Propyl, iso-Propyl, n-Butyl, iso-Butyl, sec-Butyl, tert.-Butyl, Cl, F oder Br;
n eine ganze Zahl im Bereich von eins bis vier, vorzugsweise eins oder zwei ist; und
R² Wasserstoff, einen linearen oder verzweigten Alkylrest mit eins bis acht Kohlenstoffatomen oder Aryl bedeutet, vorzugsweise einen Alkylrest mit eins bis vier Kohlenstoffatomen, besonders bevorzugt Methyl, Ethyl, n-Propyl, iso-Propyl, n-Butyl, iso-Butyl, sec-Butyl oder tert.-Butyl.

Es können jedoch auch Verbindungen mit 1,4-Benzochinon als Stammverbindung eingesetzt werden. Diese lassen sich mit der Formel (III) beschreiben 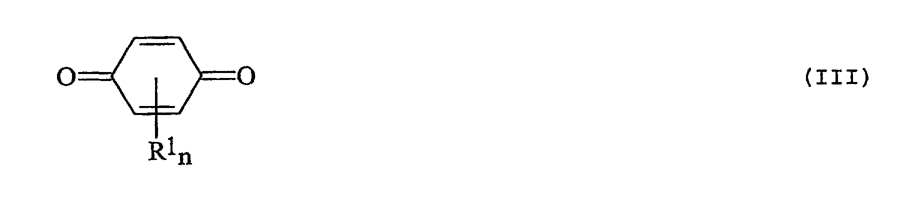 worin
R¹ einen linearen oder verzweigten Alkylrest mit eins bis acht Kohlenstoffatomen, Halogen oder Aryl bedeutet, vorzugsweise einen Alkylrest mit eins bis vier Kohlenstoffatomen, besonders bevorzugt Methyl, Ethyl, n-Propyl, iso-Propyl, n-Butyl, iso-Butyl, sec-Butyl, tert.-Butyl, Cl, F oder Br; und
n eine ganze Zahl im Bereich von eins bis vier, vorzugsweise eins oder zwei ist.

Ebenso werden Phenole der allgemeinen Struktur (IV) eingesetzt 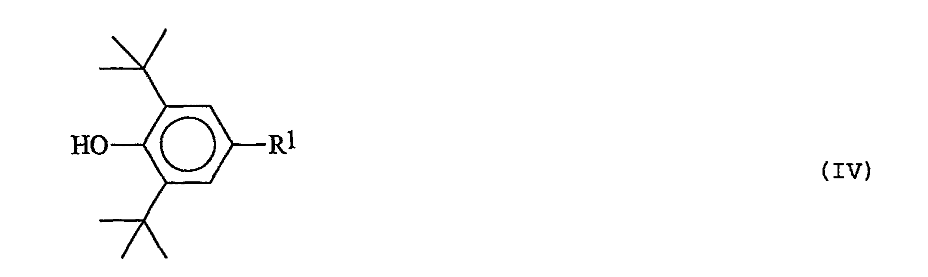 worin
R¹ einen linearen oder verzweigten Alkylrest mit eins bis acht Kohlenstoffatomen, Aryl oder Aralkyl, Proprionsäureester mit 1 bis 4 wertigen Alkoholen, welche auch Heteroatome wie S, O und N enthalten können, vorzugsweise einen Alkylrest mit eins bis vier Kohlenstoffatomen, besonders bevorzugt Methyl, Ethyl, n-Propyl, iso-Propyl, n-Butyl, iso-Butyl, sec-Butyl, tert.-Butyl, bedeutet.

Eine weitere vorteilhafte Substanzklasse stellen sterisch gehinderte Phenole auf Basis von Triazinderivaten der Formel (V) dar 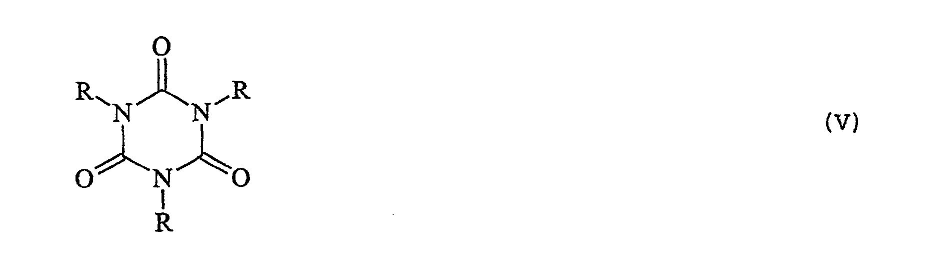 mit R = Verbindung der Formel (VI) 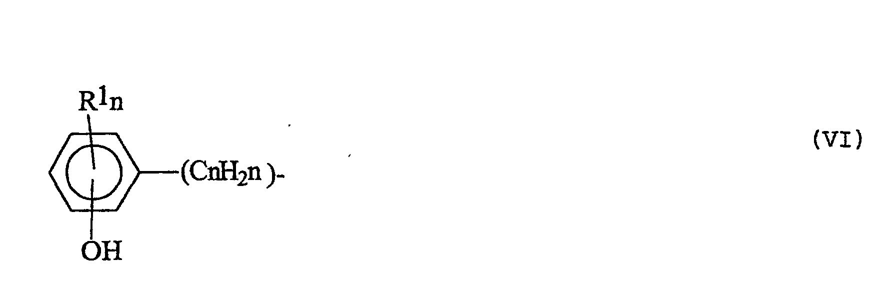 worin
R¹ = CₙH₂ₙ₊₁
mit n = 1 oder 2 ist.

Eine weitere Gruppe von bekannten Inhibitoren und Antioxidantien sind Amine, insbesondere sterisch gehinderte Amine.

Zu diesen gehören insbesondere Phenylendiamine, die durch Formel (VII) darstellbar sind 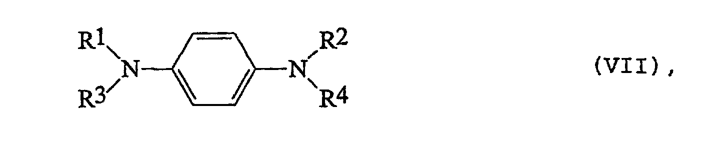 worin R¹, R², R³ und R⁴ unabhängig Wasserstoff sowie Alkyl-, Aryl-, Alkaryl-, Aralkyl-Reste mit jeweils bis zu 40, vorzugsweise bis zu 20 Kohlenstoffatomen darstellen, wobei vorzugsweise mindestens einer der Reste R¹, R², R³ und R⁴ Wasserstoff ist.

Beispielhafte p-Phenylendiamine umfassen p-Phenylendiamin worin die Reste R¹, R², R³ und R⁴ Wasserstoff sind;
N-Phenyl-N'-alkyl-p-phenylendiamine beispielsweise, N-Phenyl-N'-methyl-p-phenylendiamin, N-Phenyl-N'-ethylp-phenylendiamin, N-Phenyl-N'-propyl-p-phenylendiamin, N-Phenyl-N'-isopropyl-p-phenylendiamin, N-Phenyl-N'n-butyl-p-phenylendiamine, N-Phenyl-N'-isobutyl-pphenylendiamin, N-Phenyl-N'-sec-butyl-p-phenylendiamin, N-Phenyl-N'-tert-butyl-p-phenylendiamin, N-Phenyl-N'-npentyl-p-phenylendiamin, N-Phenyl-N'-n-hexyl-pphenylendiamin, N-Phenyl-N'-(1-methylhexyl) -pphenylendiamin, N-Phenyl-N'-(1,3-dimethylbutyl)-pphenylendiamin, N-Phenyl-N'-(1,4-dimethyl-pentyl)-pphenylendiamin;
N-Phenyl-N',N'-dialkyl-p-phenylendiamine, wie beispielsweise N-Phenyl-N',N'-dimethyl-p-phenylendiamin, N-Phenyl-N',N'-diethyl-p-phenylendiamin, N-Phenyl-N',N'-di-n-butyl-p-phenylendiamin N-Phenyl-N',N'-di-sec-butyl-p-phenylendiamin, N-Phenyl-N'methyl-N'-ethyl-p-phenylendiamin;
N,N-Dialkyl-p-phenylendiamine, wie beispielsweise N,N-Dimethyl-p-phenylendiamin und N,N'-Diethylp-phenylendiamin;
N,N'-Dialkyl-p-phenylendiamine, wie beispielsweise N,N'-Diisopropyl-p-phenylendiamin, N,N'-Diisobutylp-phenylendiamin;
N,N'-Diaryl-phenylendiamine, wie beispielsweise N,N'-Diphenyl-p-phenylendiamin;
N,N,N'-Trialkyl-p-phenylendiamine, wie beispielsweise N,N,N'-Trimethyl-p-phenylendiamin, N,N,N'-Triethylp-phenylendiamin.

Darüber hinaus bilden Phenazin-Farbstoffe eine weitere bevorzugte Gruppe. Diese umfassen insbesondere Indulin und Nigrosin. Nigrosin entsteht durch Erhitzen von Nitrobenzol, Anilin und salzsaurem Anilin mit metallischem Eisen und FeCl₃. Hierbei sind alkohollösliche Anilinfarbstoffe bevorzugt, die beispielsweise 5 Benzolkerne umfassen können, wie Dianilido-N,N-diphenylphenosafranin. Diese Stoffe sind weithin bekannt und können kommerziell erhalten werden.

Besonders erfolgreich werden die Verbindungen 1,4-Dihydroxybenzol, 4-Methoxyphenol, 2,5-Dichloro-3,6-dihydroxy-1,4-benzochinon, 1,3,5-Trimethyl-2,4,6-tris-(3,5-di-tert.butyl-4-hydroxybenzyl)benzol, 2,6-Di-tert. butyl-4-methylphenol, 2,4-Dimethyl-6-tert. butylphenol, 2,2-Bis [3,5-Bis(1,1-dimethylethyl)-4-hydroxyphenyl-1-oxoperopoxymethyl)]1,3-propandiylester, 2,2'-Thiodiethylbis-[3- (3,5-di-tert.butyl-4-hydroxyphenyl)]propionat, Octadecyl-3-(3,5-ditert.butyl-4-hydroxyphenyl)propionat, 3,5-Bis(1,1-dimethylethyl-2,2-Methylenbis-(4-methyl-6-tert.butyl)phenol, Tris-(4-tert.butyl-3-hydroxy-2,6-dimethylbenzyl)-s-triazin-2,4,6-(1H,3H,5H)trion, Tris (3,5-ditert.butyl-4-hydroxy)-s-triazin-2,4,6-(1H,3H,5H) trion, tert.-Butyl-3,5-dihydroxybenzol oder Diphenyl-p-phenylendiamin (DPPD) zusätzlich zu N,N-Diethylhydroxylamin und N-Nitroso-N-phenylhydroxylamin eingesetzt.

Je nach Zweck kann auf den Einsatz weiterer Inhibitoren verzichtet werden. Bezogen auf das Gewicht der gesamten Zusammensetzung beträgt der Anteil der zusätzlichen Inhibitoren einzeln oder als Mischung für viele Anwendungen der erfindungsgemäßen Zusammensetzung jedoch 0,01 - 0,5 % (wt/wt).

Viele dieser Inhibitoren sind kommerziell erhältlich.

Die erfindungsgemäß stabilisierte . Monomerzusammensetzungen kann weitere Bestandteile enthalten. Hierzu gehören Lösungsmittel, wie beispielsweise Benzol, Toluol, n-Hexan, Cyclohexan, Methylisobutylketon sowie Methylethylketon.

Des weiteren sind bekannte Zusatzstoffe, wie Antibindemittel, Antistatika, Antioxidantien, Biostabilisatoren, chemische Treibmittel, Entformungsmittel, Flammschutzmittel, Schmiermittel, Farbmittel, Gießverbesserungsmittel, Füllstoffe, Gleitmittel, Haftvermittler, Katalysatoren, Lichtschutzmittel, optische Aufheller, organische Phosphorverbindungen, Öle, Pigmente, Schlagzähigkeitsverbesserer, Verstärkungsmittel, Verstärkungsfasern, Verwitterungsschutzmittel und Weichmacher bevorzugte Komponenten der erfindungsgemäßen Zusammensetzung.

Die erfindungsgemäßen Zusammensetzungen können durch Mischen von mindestens einem ethylenisch ungesättigten Monomer und einer stabilisierend wirkenden Menge einer Kombination von N,N-Diethylhydroxylamin und N-Nitroso-N-Phenylhydroxylamin erhalten werden. Weitere, allgemein bekannte Inhibitoren und Antioxidantien, wie beispielsweise Hydrochinonmonomethylether und DPPD, können hierbei ebenfalls zugegeben werden.

Die erfindungsgemäßen Zusammensetzungen finden ihre Verwendung beispielsweise bei der Aufreinigung von Reaktionsmischungen. Es hat sich gezeigt, daß durch Zusatz einer Kombination von N,N-Diethylhydroxylamin und N-Nitroso-N-Phenylhydroxylamin eine sehr wirksame Stabilisierung während einer thermischen Belastung, die bei der Aufreinigung durch Destillation auftreten kann, erzielt wird.

Des weiteren sind die erfindungsgemäßen Zusammensetzungen insbesondere dazu geeignet, mit ihnen 2-Hydroxyalkyl(meth)acrylate zu erzeugen. Durch die Inhibierung einer unerwünschten Polymerisation mit Hilfe der erfindungsgemäßen Kombination aus N,N-Diethylhydroxylamin und N-Nitroso-N-phenylhydroxylamin werden sowohl die (Meth)acrylsäurereaktanten als auch die hieraus durch Umsetzung, beispielsweise mit Oxiranen, erhaltenen Hydroxyalkyl(meth)acrylate an einer radikalischen Polymerisation gehindert.

Beschrieben sind diese Verfahren zur Herstellung von Hydroxyalkyl(meth)acrylaten, bei denen eine Oxiranverbindung mit (Meth)acrylsäure in Gegenwart einer wirksamen Menge eines Katalysators und mindestens einer inhibierend wirkenden Verbindung umgesetzt wird, beispielsweise in EP-A-0 134 133, GB-PS 1 195 485, DE-PS 1 911 447 und JP-A-93-293 285.

Zu den Oxiranverbindungen, die auch als Epoxide bezeichnet werden, gehören unter anderem Ethylenoxid, Propylenoxid, 1,2-Butylenoxid und/oder 2,3-Butylenoxid, Cyclohexenoxid, Styroloxid, 1,2,3,4-Diepoxybutan, 1,2,5,6-Diepoxyhexan, Epichlorhydrin und Glycidylester. Diese Verbindungen können sowohl einzeln als auch als Mischung verwendet werden.

Bevorzugte Katalysatoren dieser Umsetzung sind beispielsweise Chrom(III)-Verbindungen, wie Chrom(III)carboxylate und Chrom(III)alkoxide. Hierzu gehören untern anderem Chrom(III)hexanoat, Chrom(III)pentanoat, Chrom(III)butyrat, Chrom(III)-2-ethylhexanoat, Chrom(III)-2-ethyldecanoat, Chrom(III)oleat, Chrom(III)stearat, Chrom(III)acrylate, Chrom(III)methacrylate, Chrom(III)benzoate und Chrom(III)acetat. Des weiteren werden auch Eisen(III)-Verbindungen, wie Eisen(III)methacrylat, zur Katalyse der zuvor genannten Umsetzung eingesetzt. Häufig werden Eisen(III)- und Chrom(III)-haltige Katalysatoren kombiniert.

Die zuvor genannten Katalysatoren und Oxiranverbindungen können im allgemeinen kommerziell erhalten werden.

Die Umsetzung von (Meth)acrylsäure mit Oxiranen findet vorzugsweise bei einer Temperatur im Bereich von 15-90°C und bei einem Druck von 1 bis 5 bar statt.

Des weiteren sind insbesondere N-Dimethylaminopropylmethacrylamid (DMAPMA) und/oder Dimethylaminoethylmethacrylat (DMAEMA) enthaltende Zusammensetzungen über lange Zeit äußerst lagerfähig, ohne daß die zugegebene Inhibitorkonzentration nicht durch Initiatoren überfahren werden könnten. Eine Abtrennung der Initiatoren vor der Polymerisation ist daher für viele Anwendungen nicht notwendig.

Die nachfolgenden Beispiele und Vergleichsbeispiele dienen zur näheren Erläuterung der vorliegenden Erfindung, ohne daß hierdurch eine Beschränkung erfolgen soll.

### Beispiel 1

### Destillation von 2-Hydroxyethylacrylat-Rohester

In einem Labordünnschichtverdampfer DS25 (Normschliff Geräte GmbH Wertheim) mit 30 cm langer verspiegelter Füllkörperkolonne (6 x 6 mm V4A-Stahlwendeln), automatischem Kolonnenkopf, Produktkühler, Intensivkühler, Vakuumeinrichtung und Schlauchpumpe wurden 6 kg 2-Hydroxyethylacrylat-Rohester kontinuierlich destilliert. Der Rohester war mit 200 ppm Hydrochinon, 600 ppm Hydrochinonmonomethylether, 200 ppm Diphenyl-p-phenylendiamin (DPPD) und 50 ppm Nigrosin (Nigrosinbase BA, Bayer AG) stabilisiert.

Der Anteil des 2-Hydroxyethylacrylats betrug gemäß GC-Analyse 84 Gew.-%. Nach Zugabe von 2,8 ml einer 85%igen wäßrigen Lösung (400 ppm) N,N-Diethylhydroxylamin und 50 ppm Kupferron (N-Nitroso-N-Phenylhydroxylamin-Ammoniumsalz) in Form einer 10%igen wäßrigen Kupferronlösung (3 ml) wird die Destillation begonnen.

Innerhalb von 8,5 Stunden wurde bei einer ProduktTemperatur von 130 °C, einem Druck im Bereich von 10 bis 12 mbar und einer Rotordrehzahl von 700 U/min keine Polymerisation beobachtet.

Es wurden 4800 g 2-Hydroxyethylacrylat mit einer durch Gaschromatographie (GC) bestimmten Reinheit von 98,6% erhalten. Das gereinigte Produkt wies eine Farbzahl gemäß APHA von 6 auf, wie diese nach DIN ISO 6271 bestimmt werden kann.

### Vergleichsbeispiel 1

Das Vergleichsbeispiel 1 wurde wie Beispiel 1 durchgeführt, außer daß nur 50 ppm Kupferron, ohne Zusatz von N,N-Diethylhydroxylamin, eingesetzt wurden.

Nach 5 Stunden trat eine Polymerisationsbildung in der Destillationskolonne auf, so daß die Destillation abgebrochen werden mußte.

Des weiteren wurde ein verminderte Ausbeute von 2430 g festgestellt. Die Farbzahl betrug 11, die Reinheit des Produkts lag bei 98,5% (GC).

### Vergleichsbeispiel 2

Vergleichsbeispiel 2 wurde durchgeführt wie Beispiel 1, außer daß zur Stabilisierung eine Mischung aus 500 ppm N,N-Diethylhydroxylamin und 500 ppm Vitamin K (2-Methyl-1,4-Naphthochinon), ohne Zusatz von Kupferron, verwendet wurde.

Hierbei war die Polymerisatbildung in der Kolonne so stark, daß der Versuch abgebrochen werden mußte, ohne daß größere Mengen an 2-Hydroxyethylacrylat isoliert werden konnten.

## Patentansprüche

1. Stabilisierte Monomerzusammensetzung, die mindestens ein ethylenisch ungesättigtes Monomer und eine stabilisierend wirkende Menge einer Kombination von N,N-Diethylhydroxylamin und N-Nitroso-N-phenylhydroxylamin aufweist.

2. Zusammensetzung gemäß Anspruch 1,daß die Zusammensetzung die Komponenten N,N-Diethylhydroxylamin und N-Nitroso-N-phenylhydroxylamin in einer synergistisch wirkenden Menge aufweist.

3. Zusammensetzung gemäß Anspruch 1 oder 2, **dadurch gekennzeichnet, daß** das Verhältnis von N,N-Diethylhydroxylamin zu N-Nitroso-N-phenylhydroxylamin im Bereich von 1:1 bis 10:1, bezogen auf das Gewicht, liegt.

4. Zusammensetzung gemäß einem oder mehreren der vorhergehenden Ansprüche, **dadurch gekennzeichnet, daß** das N,N-Diethylhydroxylamin in einer Menge im Bereich von 10 bis 500 ppm und das N-Nitroso-N-phenylhydroxylamin in einer Menge im Bereich von 10 bis 500 ppm, jeweils bezogen auf das Gesamtgewicht der Zusammensetzung, vorhanden ist.

5. Zusammensetzung gemäß einem oder mehreren der vorhergehenden Ansprüche, **dadurch gekennzeichnet, daß** die Zusammensetzung weitere Inhibitoren und Antioxidantien aufweist.

6. Zusammensetzung gemäß einem oder mehreren der vorhergehenden Ansprüche, **dadurch gekennzeichnet, daß** das ethylenisch ungesättigte Monomer ein Derivat der (Meth)acrylsäure ist.

7. Zusammensetzung gemäß Anspruch 5, **dadurch gekennzeichnet, daß** das Derivat der (Meth)acrylsäure N,N-Dimethylaminopropylmethacrylamid (DMAPMA), N,N-Dimethylaminoethylmethacrylamid (DMAEMA) und/oder Hydroxyalkyl(meth)acrylat ist.

8. Zusammensetzung gemäß einem oder mehreren der vorhergehenden Ansprüche, **dadurch gekennzeichnet, daß** die Zusammensetzung zusätzlich ein Lösungsmittel aufweist.

9. Verfahren zur Herstellung einer Zusammensetzung gemäß einem oder mehreren der Ansprüche 1 bis 8, **dadurch gekennzeichnet, daß** man mindestens ein ethylenisch ungesättigtes Monomer und eine stabilisierend wirkende Menge einer Kombination von N,N-Diethylhydroxylamin und N-Nitroso-N-phenylhydroxylamin mischt.

## Claims

1. Stabilised monomer composition which comprises at least one ethylenically unsaturated monomer and a quantity of a combination of N,N-diethylhydroxylamine and N-nitroso-N-phenylhydroxylamine with a stabilising effect.

2. Composition according to claim 1, **characterised in that** the composition contains the components N,N-diethylhydroxylamine and N-nitroso-N-phenylhydroxylamine in a quantity having a synergistic effect.

3. Composition according to claim 1 or 2, **characterised in that** the ratio of N,N-diethylhydroxylamine to N-nitroso-N-phenylhydroxylamine is in the range from 1:2 to 120:1, based on the weight.

4. Composition according to one or more of the preceding claims, **characterised in that** the N,N-diethylhydroxylamine is present in an amount in the range from 10 to 500 ppm and the N-nitroso-N-phenylhydroxylamine is present in an amount in the range from 10 to 500 ppm, based on the total weight of the composition in each case.

5. Composition according to one or more of the preceding claims, **characterised in that** the composition contains further inhibitors and antioxidants.

6. Composition according to one or more of the preceding claims, **characterised in that** the ethylenically unsaturated monomer is a derivative of (math)acrylic acid.

7. Composition according to claim 5, **characterised in that** the derivative of (meth)acrylic acid is N,N-dimethylaminopropyl methacrylamide (DMAPMA), N,N-dimethylaminoethyl methacrylamide (DMAEMA) and/or hydroxyalkyl (meth)acrylate.

8. Composition according to one or more of the preceding claims, **characterised in that** the composition additionally contains a solvent.

9. Process for preparing a composition according to one or more of claims 1 to 8, **characterised in that** at least one ethylenically unsaturated monomer and a quantity of a combination of N,N-diethylhydroxylamine and N-nitroso-N-phenylhydroxylamine with a stabilising effect are mixed together.

## Revendications

1. Composition stabilisée de monomère qui comporte au moins un monomère à insaturation éthylénique et une quantité à effet stabilisant d'une association de N,N-diéthylhydroxylamine et N-nitroso-N-phénylhydroxylamine.

2. Composition selon la revendication 1,
**caractérisée en ce que**
la composition comporte les composants N,N-diéthylhydroxylamine et N-nitroso-N-phénylhydroxylamine en une quantité agissant de façon synergique.

3. Composition selon la revendication 1 ou 2,
**caractérisée en ce que**
le rapport de la N,N-diéthylhydroxylamine à la N-nitroso-N-phényl hydroxylamine se situe dans la plage de 1:1 à 10:1. par rapport au poids.

4. Composition selon l'une quelconque des revendications précédentes,
**caractérisée en ce que**
la N,N-diéthylhydroxylamine est présente en une quantité dans la plage de 10 à 500 ppm et la N-nitroso-N-phénylhydroxylamine est présente en une quantité dans la plage de 10 à 500 ppm, dans chaque cas par rapport au poids total de la composition.

5. Composition selon l'une quelconque des revendications précédentes,
**caractérisée en ce que**
la composition comporte d'autres inhibiteurs et antioxydants.

6. Composition selon l'une quelconque des revendications précédentes,
**caractérisée en ce que**
le monomère à insaturation éthylénique est un dérivé de l'acide (méth)acrylique.

7. Composition selon la revendication 5,
**caractérisée en ce que**
le dérivé de l'acide (méth)acrylique est le N,N-diméthylaminopropylméthacrylamide (DMAPMA), le N,N-diméthylammoéthylméthacrylamide (DMAEMA) et/ou le (méth)acrylate d'hydroxyalkyle.

8. Composition selon une ou plusieurs des revendications précédentes,
**caractérisée en ce que**
la composition comporte en outre un solvant.

9. Procédé pour la préparation d'une composition selon une ou plusieurs des revendications 1 à 8,
**caractérisé en ce qu'**
on mélange au moins un monomère à insaturation éthylénique et une quantité à effet stabilisant d'une association de N,N-diéthylhydroxylamine et N-nitroso-N-phénylhydroxylamine.
